# EUROPÄISCHE PATENTSCHRIFT

(11) **EP 2 579 862 B1**
(45) Veröffentlichungstag und Bekanntmachung des Hinweises auf die Patenterteilung: **17.12.2014**
(21) Anmeldenummer: 11723222.3
(22) Anmeldetag: 31.05.2011
(51) Int. Cl.: A61K 9/00, A61K 31/01, A61K 31/05, A61K 31/12, A61K 36/185, A61K 36/63, A61K 36/81, A61K 36/87, A61P 11/02, A61P 17/18, A61P 31/12

(54) **INTRANASALE PHARMAZEUTISCHE ZUSAMMENSETZUNG ENTHALTEND OLIVENEXTRAKT (HYDROXYTYROSOL) UND HOPFENEXTRAKT (XANTHOHUMOL)**
PHARMACEUTICAL COMPOSITION WITH OLIVE EXTRACT (HYDROXYTYROSOL) AND HOP EXTRACT (XANTHOHUMOL) FOR INTRANASAL APPLICATION
COMPOSITION PHARMACEUTIQUE INTRANASALE CONTENANT UN EXTRAIT D'OLIVE (HYDROXYTYROSOL) ET UN EXTRAIT DE HOUBLON (XANTHOHUMOL)

(30) Priorität: 09.06.2010 CH 9212010
(43) Veröffentlichungstag der Anmeldung: 17.04.2013
(73) Patentinhaber: Weluga-Pharm Anstalt, 9485 Nendeln (LI)
(72) Erfinder: EHRENBERGER, Klaus, A-1090 Wien (AT); BIEBERSCHULTE,Werner, FL-9492 Eschen (LI)
(74) Vertreter: Hasler, Erich
(86) Internationale Anmeldenummer: PCT/CH2011/000128
(87) Internationale Veröffentlichungsnummer: WO 2011/153647

(56) Entgegenhaltungen:
- WO-A1-2009/024317
- DE-U1- 20 203 549
- US-B1- 6 391 346
- SAEDISOMEOLIA A ET AL: "Lycopene enrichment of cultured airway epithelial cells decreases the inflammation induced by rhinovirus infection and lipopolysaccharide", JOURNAL OF NUTRITIONAL BIOCHEMISTRY, BUTTERWORTH PUBLISHERS, STONEHAM, GB LNKD- DOI:10.1016/J.JNUTBIO.2008.06.001, Bd. 20, Nr. 8, 1. August 2009 (2009-08-01) , Seiten 577-585, XP026283513, ISSN: 0955-2863 [gefunden am 2008-09-27]
- Buhler D, Miranda C: "Antioxidant Activities of Flavonoids", , November 2000 (2000-11), XP002593774, Gefunden im Internet: URL:http://lpi.oregonstate.edu/f-w00/flavo noid.html [gefunden am 2010-07-26]

## Beschreibung

### Technisches Gebiet

Die vorliegende Erfindung betrifft eine pharmazeutische Zusammensetzung mit Antioxidantien, insbesondere zur topischen Behandlung der Symptome bei viralem, allergischem und vasomotorischem Schnupfen.
Die Zusammensetzung führt zur Befreiung der Nasenatmung durch Schleimhautabschwellung und Reduktion der Sekretproduktion.

### Stand der Technik

Schnupfen ist eine akute oder chronische Entzündung der Nasenschleimhaut, die verschiedene Ursachen haben kann.

Beim viralen Schnupfen reagiert die Nasenschleimhaut auf Viren, die sich auf der Oberfläche der Schleimhaut niederlassen und eine Immunreaktion auslösen. Durch vermehrte Schleim- und Flüssigkeitsproduktion wird versucht, die Eindringlinge wegzuspülen. Gleichzeitig werden durch verstärkte Durchblutung - einhergehend mit einem Anschwellen der Schleimhaut - Immunzellen herbeigerufen, die die eindringenden Viren unschädlich machen sollen. Ein viraler Schnupfen kann das Immunsystem stark beanspruchen und den Körper massiv schwächen. Zumeist schädigen die Viren die Oberfläche der Schleimhaut, so dass eine tagelange Entzündung die Folge ist. Es ist bekannt, dass bei Entzündungsprozessen Sauerstoffradikale (ROS; "reactive oxigen species") eine wesentliche Rolle spielen.

Ist die Nasenschleimhaut ständig gereizt oder nachhaltig geschädigt, funktioniert sie nicht mehr normal, sondern produziert ständig zu viel Flüssigkeit und Schleim. Bei der sogenannten Rhinitis vasomotorica (Vasomotorischer Schnupfen) oder auch Fliessschnupfen läuft die Nase andauernd. Ein erkennbarer Grund ist häufig nicht vorhanden, aber bei leichten Reizungen wie kalte oder trockene Luft beginnt die Nase stark zu laufen und manchmal auch anzuschwellen. Nasenspülungen und Befeuchtungen können helfen, aber oft muss die zugrunde liegende Entzündung mit Kortisonsprays behandelt werden. Wie beim viralen Schnupfen erwähnt, spielen bei Entzündungsprozessen Sauerstoffradikale bekanntermassen eine Rolle.

Allergische Erkrankungen wie Asthma, Rhinitis oder Ekzeme nehmen epidemische Formen an, sowohl in Industrie- als auch in Entwicklungsländern. Die Anzahl Patienten mit saisonaler allergischer Rhinitis nimmt jährlich zu. Die Rhinitis allergica (Heuschnupfen) ist die häufigste Immunerkrankung und befällt ca. 30% der Erwachsenen und bis zu 40% der Kinder industrialisierter Gesellschaften. Die saisonale allergische Rhinitis wird im Frühling oder Sommer durch Baum-, Gräser- und Unkrautpollen ausgelöst, während die chronische Allergie eher auf Hausallergene, wie Hausstaubmilbe, Schimmelpilze und Tierhaare, sowie auf Umweltfaktoren wie Feinstaub und Ozon zurückgeht.
Die allergischen Erkrankungen werden üblicherweise mit Antihistaminen, Corticosteroiden und weiteren chemischen Substanzen behandelt. Diese Medikamente besitzen jedoch Nebenwirkungen, vor allem wenn sie über einen längeren Zeitraum eingenommen werden müssen, was bei allergischen Erkrankungen der Fall ist.

Zur Pathophysiologie ist zu sagen, dass Umweltallergene mittels Oberflächenproteinen T-Zell-Lymphozyten der Nasenschleimhaut stimulieren, die ihrerseits über Interleukine als Signalmoleküle basophile, eosinophile, B- und Mast-Zellen aktivieren. Allergiker haben eine genetisch bedingte, erhöhte Produktionsrate von Immunglobulin E (IgE) - Antikörpern gegen Allergene. IgE hat eine hohe Affinität zu spezifischen Rezeptoren der Mast-Zellen der Nasenschleimhaut. Dieser Kontakt ist das Signal für die Ausschüttung einer Reihe von Mediatoren und Zytokinen, die - individuell unterschiedlich - sofort oder verzögert lokale, klinisch manifeste Entzündungszeichen auslösen und sekundär eine leukozytäre Infiltration der Nasenschleimhaut zur Folge haben. Auf Kenntnis dieser immunologischen Zusammenhänge beruhen auch die klassischen Therapieansätze: Mediatorenhemmer (Antihistaminika), Mast-Zell-Stabilisatoren (Cromoglukate), Allergen-spezifische Immuntherapie (Desensibilisierung), AntiIgE-Therapie, Corticoide.

In den letzten Jahren verdichteten sich die Hinweise, dass ein Überschuss an freien Sauerstoffradikalen (ROS) als treibender Motor der einzelnen Schritte der Pathogenese der allergischen Rhinitis anzusehen ist. Pollenkörner produzieren enzymatisch bereits beim Erstkontakt mit der Nasenschleimhaut innerhalb weniger Minuten grosse Mengen an ROS. Es ist bekannt, dass vor allem eosinophile Leukozyten, die häufigsten Effektorzellen der allergischen Rhinitis, durch das von den Mast-Zellen ausgeschüttete Signalmolekül Eotaxin angeregt werden, überschiessend ROS freizusetzen. Diese sekundäre ROS Produktion kurbelt die allergisch-entzündliche Reaktion der Schleimhaut weiter an, reduziert dramatisch die ziliare Schlagfrequenz und fördert die Chronifizierung des Leidens durch die sekundäre Bildung von Peroxynitrit (RNS; "reactive nitrogen species"), einem aggressiven Reaktionsprodukt von ROS mit dem körpereigenen Signalstoff NO (Stickstoffmonoxyd; "nitric oxide").

Die überschiessende Freisetzung aggressiver ROS als Immunantwort von Allergikern auf Allergene führte konsequenterweise zur Entwicklung alternativer Therapieansätze. Ziel ist es, unabhängig vom Typ des Allergens, also unspezifisch, allergische Reaktionen durch den Einsatz von Antioxidantien zu unterdrücken.

Es ist bekannt, dass Pflanzenextrakte verschiedene Antioxidantien enthalten. Diese zeigen ein breites Spektrum an Bioaktivitäten. Gemäss Literatur können diese Extrakte für die unterstützende Behandlung verschiedener Krankheiten eingesetzt werden, welche in Zusammenhang mit oxydativem Stress stehen. Erwähnte Krankheiten sind z.B. Krebs, Diabetes, Hauterkrankungen, entzündliche Erkrankungen, virale und bakterielle Infektionen oder Asthma. Die Anwendung der Pflanzenextrakte erfolgt vor allem in oraler Form. Dadurch ist eine relativ hohe Dosierung erforderlich und es muss über einen längeren Zeitraum therapiert werden, um eine entsprechende Wirkung zu erzielen. Tatsächlich erweisen sich antioxidativ wirksame Pflanzenextrakte, die vielfach aus der traditionellen asiatischen Medizin her bekannt sind, als zuverlässige Antiallergika. Allerdings wirken diese Phytopharmaka erst nach längerer oraler Einnahme. Saisonale, akute allergische Attacken werden auf diese Weise kaum beeinflusst.

Bei Entzündungen der Nasenschleimhaut können ROS bzw. RNS als Teil unterschiedlichster Wirkmechanismen eine Rolle spielen. Daher besteht eine Aufgabe der vorliegenden Erfindung darin, eine pharmazeutische Zusammensetzung bereitzustellen, welche bei einem möglichst grossen Teil der pathophysiologischen Mechanismen antagonistisch wirkt. Weitere Aufgaben und Vorteile ergeben sich aus der nachfolgenden Beschreibung.

DE 202 03 549 beschreibt synergistische intranasale Zusammensetzungen, enthaltend Olivenöl und Hippophae-rhamnoides-Öl zur therapeutischen Behandlung von Rhinopathien.

### Darstellung der Erfindung

Da die Mechanismen, welche unter Mitwirkung von ROS bzw. RNS an der Entstehung von Entzündungen der Nasenschleimhaut beteiligt sind, nicht alle durch eine einzige pflanzliche Molekülstruktur antagonisiert werden können, wurde der Ansatz verfolgt, spezifisch ausgewählte Moleküle zu kombinieren.

Es wurde gefunden, dass eine pharmazeutische Zusammensetzung beinhaltend als Wirkstoffe Hydroxytyrosol und Xanthohumol, bei Entzündungen der Nasenschleimhaut eine überraschend starke, positive Wirkung entfaltet. Unter einem "Wirkstoff" ist hierbei bevorzugt ein Stoff zu verstehen, der in pharmazeutisch wirksamer Konzentration vorliegt und vorzugsweise zum Zwecke einer pharmazeutischen Wirkung beigegeben wird.

Die genannte Zusammensetzung ist deshalb insbesondere zur prophylaktischen und/oder kurativen Behandlung von Schnupfen und besonders zur Anwendung bei viralem, allergischem oder vasomotorischem Schnupfen geeignet.

Vorteilhaft ist eine Zusammensetzung wie sie in diesem Dokument beschrieben wird zur Verwendung als Arzneimittel, insbesondere zur Behandlung von Schnupfen, vorzugsweise viralem, allergischem oder vasomotorischem Schnupfen. Weiterhin ist die Verwendung der Zusammensetzung wie sie in diesem Dokument beschrieben wird zur Herstellung eines Arzneimittels bevorzugt, insbesondere zur beschriebenen Behandlung von Schnupfen.

Die Zusammensetzung soll mit Vorteil auch für die Anwendung bei Kindern und/ oder Kleinkindern geeignet sein. Sie ist zu diesem Zweck vorzugsweise frei oder im Wesentlichen frei von resorbierbaren, vasoaktiven Stoffen.

Es ist wünschenswert, mit der erfindungsgemässen Zusammensetzung eine möglichst lange Wirkungsdauer und eine kurze Behandlungszeit zu erreichen. Es wurde festgestellt, dass dies durch eine topische Anwendung, insbesondere in der Nase, bewerkstelligt werden kann. Besonders bevorzugt liegt die Zusammensetzung deshalb in topisch anzuwendender Form vor, und zwar mit Vorteil in der Form einer flüssigen Formulierung und/oder in der Form einer Formulierung für die intranasale Applikation (z.B. Tropfen, Lösung, Spray, Dosierspray). Besonders bevorzugt sind ein Nasenspray, Nasentropfen oder eine Nasencreme, denn es wurde festgestellt, dass lokal in die Nase applizierte Antioxidantien einen raschen, stark dämpfenden Effekt auf die Symptome insbesondere der allergischen Rhinitis aber auch der anderen Arten von Schnupfen, wie sie beschrieben wurden, haben.

Bei der Auswahl geeigneter Moleküle ist zu beachten, dass Entzündungen der Nasenschleimhaut durch eine Fülle unterschiedlicher ROS/RNS-Aktionen ausgelöst werden, die nicht alle durch eine einzige Molekülstruktur antagonisiert werden. Daher ist eine Kombination selektiv ausgewählter Wirkstoffe anzustreben, die das gesamte Spektrum der pathophysiologischen ROS/RNS-Aktionen - oder zumindest einen wesentlichen Teil davon - in der entzündeten Nasenschleimhaut antagonisiert. Dies wird durch eine Kombination aus Hydroxytyrosol und Xanthohumol erreicht.

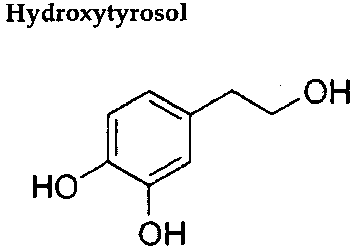

Hydroxytyrosol ist z.B. Bestandteil des kalt-gepressten Olivenöls. Die Orthodiphenolstruktur ist Voraussetzung für eine starke antioxidative Aktivität gegen die ROS-Komponenten Peroxydradikale, Hydroxylradikale und vor allem gegen Peroxinitrite (RNS). Auf diese Weise verhindert Hydroxytyrosol die Chronifizierung der Nasenschleimhautentzündung. Hydroxytyrosol besitzt den höchsten ORAC Wert, welcher die Antioxidantienkapazität eines Moleküls beschreibt.

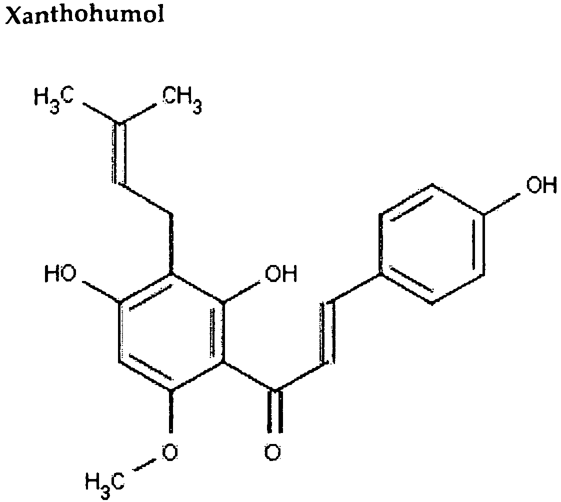

Xanthohumol ist ein stark antioxydativ wirkendes Polyphenol, das z.B. in Hopfenextrakt zu finden ist. Wie in der Literatur beschrieben wird, blockiert es die ROS-induzierte Freisetzung von Mediatoren, inklusive Histamin, aus basophilen und Mastzellen. Im Gegensatz dazu besetzen therapeutische Antihistaminika als "klassische" Mediatorenhemmer die Histamin-spezifischen Rezeptoren der Nasenschleimhaut.

Xanthohumol verhindert also die ROS-induzierte Ausschüttung der Mediatoren wohingegen Hydroxytyrosol bereits freigesetzte ROS-Radikale abfängt. Erfindungsgemäss werden also zwei spezielle Antioxidantien mit unterschiedlichem Wirkprinzip kombiniert. Deren Kombination führt zu einer synergistischen Wirkung, die den zu erwartenden Effekt einer Kombination zweier beliebiger Antioxidantien wesentlich übersteigt. Tatsächlich können mittels einer pharmazeutischen Zusammensetzung mit den genannten Wirkstoffen sowohl viraler als auch vasomotorischer und allergischer Schnupfen behandelt werden.

Es wurde weiterhin gefunden, dass eine erfindungsgemässe Zusammensetzung dann besonders vorteilhaft ist, wenn sie zusätzlich als Wirkstoffe Resveratrol und/oder Lycopin beinhaltet, wobei Resveratrol besonders bevorzugt ist.

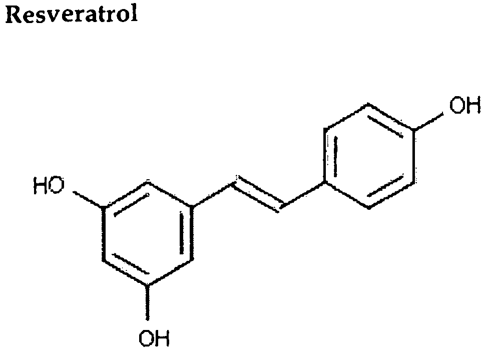

Resveratrol findet sich als Phytoalexin in der Schale und den Kernen roter Weintrauben und somit im Rotwein selbst. Es antagonisiert zuverlässig die Superoxid-Radikal-Komponente von ROS und wirkt darüber hinaus bekanntermassen allgemein entzündungshemmend durch Hemmung der Prostaglandin-Produktion und der Cyclooxygenase-2-Aktivität.

Lycopin ist ebenfalls ein Radikalfänger, insbesondere von Peroxylradikalen und Peroxynitrit. Es wird auch als Quencher für Singulettsauerstoff beschrieben. Dieser wird z.B. durch photochemische Reaktion bei der Lichtabsorbtion gebildet und ist hochreaktiv. Er kann verschiedene Aminosäuren in Proteinen, Nukleinsäuren sowie ungesättigte Fettsäuren oxidieren. Bei dieser Quenchingreaktion wird Lycopin chemisch nicht umgewandelt und steht für weitere Quenchingprozesse zur Verfügung. Die Quenchingrate von Lycopin ist etwa 100mal so gross wie bei alpha-Tocopherol.

Die besondere Vorteilhaftigkeit des Vorhandenseins von Resveratrol und Lycopin begründet sich darin, dass ein noch weiteres Spektrum der pathophysiologischen Mechanismen, bei denen ROS bzw. RNS involviert sind, in der entzündeten Nasenschleimhaut antagonisiert wird.

Zusammenfassend ist zu sagen, dass die Kombination aus Hydroxytyrosol, Xanthohumol und bevorzugt auch Resveratrol und/ oder Lycopin über die Beeinflussung einer Vielzahl von Wirkmechanismen zu einer überraschend starken Verminderung der ROS- bzw. RNS-abhängigen Schädigung der Nasenschleimhaut führt, und zwar sowohl bei viralem als auch bei vasomotorischem oder allergischem Schnupfen (insbesondere Typ I Allergien). Dies gilt in besonderem Masse für die topische Anwendung der Wirkstoffkombination.

Vorzugsweise sind unter den Bezeichnungen der entsprechenden Wirkstoffe (Xanthohumol, Hydroxytyrosol, Resveratrol, Lycopin) auch solche Stoffe zu verstehen, die im Körper in den jeweiligen Wirkstoff und/oder in seine biologisch aktive Form umgewandelt werden.

Nach einer bevorzugten Ausführungsform sind ein oder mehrere der genannten Wirkstoffe in der erfindungsgemässen Zusammensetzung ganz oder teilweise biogen. Mit Vorteil sind alle genannten Wirkstoffe in der pharmazeutischen Zusammensetzung biogen und zwar vorzugsweise vollständig oder im Wesentlichen vollständig.

Gemäss einer weiteren bevorzugten Ausführungsform sollen die ausgewählten und kombinierten, antioxydativ wirksamen Moleküle bzw. Wirkstoffe Bestandteile von Nutzpflanzen sein bzw. aus diesen gewonnen werden. Deren gute Verträglichkeit ist durch den jahrtausendelangen Gebrauch belegt. Auf diese Weise kann die Gefahr von Nebenwirkungen gering gehalten werden. Ausserdem kann eine gute Verträglichkeit so auch erreicht werden, ohne dass die Wirkstoffe in hoher Reinheit zur Verfügung gestellt werden müssen.

Mit Vorteil zeichnet sich die erfindungsgemässe Zusammensetzung dadurch aus, dass einer oder mehrere und vorzugsweise alle der genannten Wirkstoffe in der Zusammensetzung (falls vorhanden) als Teil von Pflanzenextrakten vorliegen bzw. ihr in der Form von Pflanzenextrakten zugegeben sind.

Die Konzentration des jeweiligen Wirkstoffs im jeweiligen Pflanzenextrakt beträgt vorzugsweise mindestens 5%, besonders bevorzugt mindestens 10% und insbesondere mindestens 20% bezogen auf das Gewicht des jeweiligen Pflanzenextrakts. Bevorzugte maximale Konzentrationen sind 50%, vorzugsweise 40% und insbesondere 30%.

Bei Angaben in Prozent mit Bezugnahme auf ein Gewicht als Referenzwert, ist von Gewichtsprozent die Rede.

Die Konzentration des jeweiligen Pflanzenextrakts in der Zusammensetzung beträgt mit Vorteil jeweils 0,05 bis 5%, besonders bevorzugt 0,08 bis 2% und insbesondere 0,1 bis 0,5% bezogen auf das Gewicht der Zusammensetzung.

Die bevorzugte Konzentration kann vom verwendeten Extrakt abhängen und es können sowohl Pflanzenextrakte als auch angereicherte Pflanzenextrakte verwendet werden. Die oben gemachten Angaben zu den Konzentrationen der Wirkstoffe in den Pflanzenextrakten bzw. der Pflanzenextrakte in der Zusammensetzung sollen deshalb für einzelne Wirkstoffe bzw. Pflanzenextrakte und für beliebige Kombinationen derselben gelten.

Bei den Pflanzenextrakten handelt es sich vorzugsweise um einen oder mehrere der folgenden: Hopfenextrakt, Olivenextrakt, Traubenextrakt (insbesondere Traubenkernextrakt), Tomatenextrakt.

Die Pflanzenextrakte können in verschiedener Form in der Zusammensetzung vorliegen und/ oder in verschiedener Form bei der Herstellung der Zusammensetzung eingesetzt oder zugegeben werden. Bevorzugt sind: Ein wässriger oder öliger Pflanzenextrakt, sowie Lösungen von standardisierten Pflanzenextrakten (z.B. standardisierter Hopfen-, Oliven-, Trauben- und/ oder Tomatenextrakt). Olivenextrakt wird bevorzugt in flüssiger Form eingesetzt, wegen des hohen Gehalts an Hydroxytyrosol und der guten Wasserlöslichkeit.

Von besonderem Interesse sind darüber hinaus liposomale Formulierungen mit obigen Extrakten oder eine Emulsion mit obigen Pflanzenextrakten, insbesondere eine Nanoemulsion. Die Zusammensetzung liegt also nach einer bevorzugten Ausgestaltungsvariante (nach Zugabe der Extrakte) als liposomale Formulierung bzw. als Emulsion vor.

Unter "liposomalen Formulierungen" sind vorzugsweise Formulierungen mit aus Lipidmembranen geformten kugeligen Partikeln in einer Größe zwischen etwa 20 und mehreren hundert Nanometern zu verstehen. Einer oder mehrere der genannten Wirkstoffe befindet sich dabei mit Vorteil ganz oder teilweise in den Partikeln. Vorzugsweise befindet sich der grössere Teil der genannten, in der Zusammensetzung vorhandenen Wirkstoffe in den Partikeln.

Unter "Nanoemulsion" seien vorzugsweise kolloiddisperse Systeme zu verstehen, die Partikelgrössen vergleichbar mit denen von Liposomen im Bereich von 20-300 Nanometern aufweisen. Die Grenzfläche der Nanoemulsion kann wie bei den Liposomen aus Phospholipiden bestehen. Einer oder mehrere der genannten Wirkstoffe befindet sich dabei mit Vorteil ganz oder teilweise in den Partikeln. Vorzugsweise befindet sich in diesem Fall der grössere Teil der genannten, in der Zusammensetzung vorhandenen Wirkstoffe in den Partikeln.

Bevorzugt werden folgende Extrakte verwendet (Gewichtsprozente):
- Hopfenextrakt z.B. aus Pellets von Saazer Hopfen. Idealerweise wird ein xanthohumolreicher Hopfenextrakt verwendet (mind. 15%, vorzugsweise mind. 20 % Xanthohumol).
- Olivenextrakt mit mind.15 %, vorzugsweise mind. 40 % Hydroxytyrosol.
- Traubenextrakt mit mind. 5 %, vorzugsweise mind.10 % Resveratrol.
- Tomatenextrakt mit mind. 5 %, vorzugsweise mind. 10 % Lycopin

Es können jeweils ein oder mehrere verschiedene der genannten Arten von Extrakten (z.B. zwei unterschiedliche Traubenkernextrakte) in der Zusammensetzung vorhanden sein. Zusätzlich dazu können weitere Pflanzenextrakte mit Antioxidantien und angereicherte Formen derselben in der Zusammensetzung vorhanden sein.

Die Extrakte können nach dem Fachmann bekannten Herstellungsverfahren in variabler Zusammensetzung mit Lösungsmitteln wie z.B. Wasser, Alkoholen (Ethanol, 2-Propanol), Aceton etc. und Gemischen davon bei Temperaturen von Raumtemperatur bis 100 °C unter gelinder bis heftiger Durchmischung oder durch Perkolation innerhalb von 10 Min. bis 48 Std. unter Normaldruck oder erhöhtem Druck erhalten werden. Zur Anreicherung von wirksamkeitsrelevanten Komponenten können weitere Konzentrierungsschritte durchgeführt werden.

Bevorzugt werden die Extrakte durch speziell entwickelte Verfahren hergestellt. Mit Vorteil werden z.B. Hopfenextrakt durch zweistufige Extraktion mit verdichtetem CO₂ und Olivenextrakt durch Heisswasserextraktion gewonnen. Hier handelt es sich um dem Fachmann bekannte und in Patenten beschriebene Verfahren. Nach einer besonders bevorzugten Ausgestaltung der vorliegenden Erfindung wird aber ein flüssiger Olivenextrakt eingesetzt.

Wie oben beschrieben, kann die Zusammensetzung als liposomale Formulierung bzw. als Emulsion vorliegen. Die Nanoemulsion bzw. die liposomale Formulierung enthält vorzugsweise ein Membran-bildendes Molekül und einen Co-Emulgator neben den erwähnten Pflanzenextrakten.

Als membranbildende Moleküle werden mit Vorteil Substanzen eingesetzt, die in der Lage sind, zweischichtige Systeme zu bilden (sogenannte Doppelschichten, insbesondere Lipiddoppelschichten). Vorzugsweise wird ein Phospholipid als membranbildendes Molekül eingesetzt, wobei es sich um ein hydriertes oder teilweise hydriertes Phospolipid handeln kann. Bevorzugt wird ein natürlich gewonnenes oder künstlich hergestelltes Lezithin verwendet. Beispielsweise kann dieses aus Sojabohnen oder Hühnereiern gewonnen werden.

Als Beispiele für bevorzugte Phospholipide sind zu nennen: Phosphatidylcholin, Phosphatidylethanolamin, Phosphatidylglycerol, Phosphatidylinositol, Phosphatidylserine und Sphingomyelin. Die Acyl-Seitenkette kann entweder gesättigt oder ungesättigt vorliegen und weist vorzugsweise 12 bis 22 und besonders bevorzugt 14 bis 18 Kohlenstoffatome auf.

Weitere liposomenbildende Membranlipide wie Glycolipide, Ceramide, Ganglioside und/oder Cerebroside können anstelle von oder teilweise anstelle von Phospholipiden verwendet werden.

Die Lipide können aus natürlichen Quellen wie Pflanzen, Tieren oder Mikroorganismen stammen bzw. gewonnen werden oder synthetisch hergestellt sein. Dazu gehören auch Monoacyl-Phospholipide, welches z.B. aus Polyethylenglycol (PEG) erhalten werden kann, beispielsweise PEGyliertes (d.h. durch Anhängen von PEG-Ketten hergestelltes) Monoacyl-Phosphatidylethanolamin.

Gemäss einer besonders bevorzugten Ausführungsform wird ein Phospholipid der nachfolgenden Formel als membranbildende Verbindung verwendet, 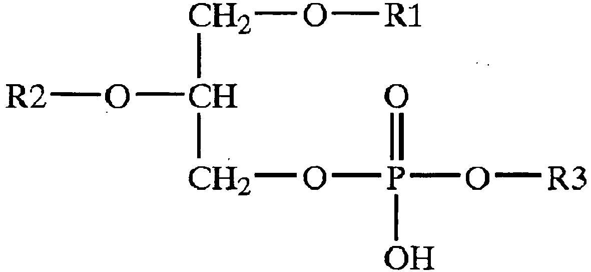 wobei
R1 = Acyl-Gruppe mit C₁₀ bis C₂₀;
R2 = Wasserstoff oder eine Acyl-Gruppe mit C₁₀ bis C₂₀;
R3 = Wasserstoff oder 2-Trimethylamino-1-ethyl oder 2-Amino-1-ethyl oder eine unsubstituierte Alkylgruppe mit C₁ bis C₅ oder eine mit einer oder mehreren Carboxyl-, Hydroxy- oder Amino-Gruppen substituierte Alkylgruppe mit C₁ bis C₅ oder eine Inositolgruppe oder eine Glyceryl-Gruppe oder Salze von den genannten Verbindungen.

Die Acyl-Gruppe mit C₁₀ bis C₂₀ ist vorzugsweise eine geradkettige Alkanoyl-Gruppe mit C₁₀ bis C₂₀ und einer geraden Anzahl C-Atome oder eine geradkettige Alkanoyl-Gruppe mit C₁₀ bis C₂₀, welche eine oder mehrere Doppelbindungen und eine gerade Anzahl C-Atome aufweist. Bei den geradkettigen Alkanoyl-Gruppen mit einer geraden Anzahl C-Atome handelt es sich z.B. um n-Dodecanoyl, n-Tetradecanoyl, n-Hexadecanoyl oder n-Octadecanoyl. Geradkettige Alkanoly-Gruppen mit C₁₀ bis C₂₀ und einer Doppelbindung, sowie einer geraden Anzahl C-Atome sind z.B. 6-cis- oder 6-trans-, 9-cis- oder 9-trans-Dodecenoyl, -Tetradecenoyl, -Hexadecenoyl, -Octadecenoyl oder -Icosenoyl, insbesondere 9-cis-Octadecenoyl (oleoyl) und 9,12-cis-Octadecadienoyl oder 9,12,15-cis-Octadecatrienoyl.

Ein Phospholipid mit der oben genannten Formel, bei dem R3 2-Trimetylamino-1-ethyl ist, wird als Lecitin bezeichnet und ein Phospholipid mit der oben genannten Formel, bei dem R3 2-Amino-1-ethyl ist, wird auch Cephalin genannt. Natürlich vorkommendes Cephalin oder Lecitin mit unterschiedlichen oder identischen Acyl-Gruppen oder Mischungen derselben sind besonders geeignet.

Die membranbildende Komponente liegt bevorzugt in einer Konzentration von 0,1 bis 30%, vorzugsweise 5 bis 10% vor, bezogen auf das Gewicht der gesamten Zusammensetzung.

Bevorzugt beinhaltet die Zusammensetzung also ein membranbildendes Molekül und einen Co-Emulgator. Die Zusammensetzung kann jedoch auch mehrere verschiedene Arten von membranbildenden Molekülen und/oder mehrere verschiedene Arten von Co-Emulgatoren beinhalten.

Einer der folgenden Co-Emulgatoren oder Mischungen aus zwei oder mehreren Co-Emulgatoren wie sie im Folgenden aufgeführt werden, können mit Vorteil als Emulgatoren eingesetzt werden: Alkalimetall-, Ammonium- oder Aminium- Salze von Fettsäuren, beispielsweise Lithium-, Natrium-, Kalium-, Ammonium-, Triethylamin-, Ethanolamin-, Diethanölamin- oder Triethanolamin-Salze. Natrium-, Kalium- oder Ammonium-(NRiR₂R3) Salze sind bevorzugt, wobei R1, R2 und R3 unabhängig voneinander mit Vorteil die folgenden Substituenten bezeichnen: Wasserstoff; Alkyl mit C₁ bis C₄ oder Hydroxyalkyl mit C₁ bis C₄; Isosorbid-Dimethylether; Alkylsulfate wie Natriumdodecylsulfat; Salze der Gallensäure, z.B. Natrium-Cholat, Natrium-Glycocholat und Natrium-Taurocholat; partielle Fettsäureester von Sorbitan, wie z.B. Sorbitan-Monolaurat; Zuckerester, wie z.B. Sucrose-Monolaurat; nur teilweise mit Fettsäuren veresterte Glyceride, wie z.B. Laurinsäure-Monoglycerid; Polyglycerolester von Fettsäuren; Propylenglycolester von Fettsäuren; Milchsäureester von Fettsäuren, wie z.B. Natrium-stearoyl-lactyl-2-lactat; Proteine, wie z.B. Casein.

Emulgatoren des Polyoxyethylen-Typs sind besonders bevorzugt. Beispiele solcher Emulgatoren sind: Polyethoxylierte (d.h. mit Einheiten aus Ethylenoxyd verbundene) Sorbitanfettsäureester, wie z.B. Polysorbat 80; polyethoxylierte Vitamin-E-Derivate, wie z.B. Vitamin-E-polyethylenglycol-1000-succinat; polyethoxylierte, teilweise mit Fettsäuren veresterte Glyceride, wie z.B. Diethylenglycol-Monostearat; polyethoxylierte Kohlenwasserstoffe; Blockpolymere von Ethylenoxid und Propylenoxid, wie z.B. Poloxamer 188.

Der Emulgator liegt in der Zusammensetzung mit Vorteil in einer Konzentration von 0,5 bis 30 %, vorzugsweise 0,75 bis 10 % und insbesondere von 1 bis 5 %, bezogen auf das Gewicht der gesamten Zusammensetzung (Gesamtgewicht) vor.

Die erfindungsgemässe Zusammensetzung kann in einer topischen Anwendung, zur Unterstützung der Behandlung oder zur Prophylaxe von viralem, vasomotorischem und allergischem Schnupfen eingesetzt werden. Sie wird bevorzugt vor Auftreten der ersten Symptome oder sofort nach Auftreten der ersten Symptome angewendet. Aber auch bei einer bereits vorhandenen Rhinitis ist die Verwendung der Zusammensetzung angezeigt. Bevorzugt wird das Mittel in Form eines Nasensprays angewendet. Das Mittel kann jedoch auch zur Unterstützung anderer Therapien verwendet werden.

Zusammenfassend sei offenbart:
1.) Eine pharmazeutische Zusammensetzung beinhaltend als Wirkstoffe Hydroxytyrosol und Xanthohumol.
2.) Eine Zusammensetzung nach Punkt 1.) zur prophylaktischen und/oder kurativen Behandlung der Symptome von Schnupfen, insbesondere von viralem, allergischem und/oder vasomotorischem Schnupfen.
3.) Eine Zusammensetzung nach einem der Punkte 1.) bis 2.), in der Form einer Formulierung für die intranasale Applikation, vorzugsweise in der Form eines Sprays.
4.) Eine Zusammensetzung nach einem der Punkte 1.) bis 3.), wobei sie zusätzlich als Wirkstoffe Resveratrol und/oder Lycopin beinhaltet.
5.) Eine Zusammensetzung nach einem der Punkte 1.) bis 4.), wobei einer oder mehrere der Wirkstoffe biogen sind.
6.) Eine Zusammensetzung nach einem der Punkte 1.) bis 5.), wobei einer oder mehrere der Wirkstoffe in der Zusammensetzung als Teil von Pflanzenextrakten vorliegen,
   - wobei die Konzentration des jeweiligen Wirkstoffs im jeweiligen Pflanzenextrakt vorzugsweise mindestens 5%, besonders bevorzugt mindestens 10% und insbesondere mindestens 20% bezogen auf das Gewicht des jeweiligen Pflanzenextrakts beträgt und
   - wobei die Konzentration des jeweiligen Pflanzenextrakts in der Zusammensetzung jeweils vorzugsweise 0,05 bis 5%, besonders bevorzugt 0,08 bis 2% und insbesondere 0,1 bis 0,5% bezogen auf das Gewicht der Zusammensetzung beträgt.
7.) Eine Zusammensetzung nach Punkt 6.), wobei es sich bei den Pflanzenextrakten um einen oder mehrere der folgenden handelt: Hopfenextrakt, Olivenextrakt, Traubenextrakt, Tomatenextrakt.
8.) Eine Zusammensetzung nach einem der Punkte 1.) bis 7.), wobei sie ein membranbildendes Molekül und einen Co Emulgator beinhaltet.
9.) Eine Zusammensetzung nach einem der Punkte 1.) bis 4.), wobei sie den Wirkstoff Hydroxytyrosol als Teil von Olivenextrakt und den Wirkstoff Xanthohumol als Teil von Hopfenextrakt enthält.
10.) Eine Zusammensetzung nach Punkt 9.), wobei der Olivenextrakt in flüssiger Form verwendet wird.
11.) Eine Zusammensetzung nach einem der Punkte 1.) bis 10.); wobei sie für die Anwendung bei Kindern und/oder Kleinkindern geeignet ist, wobei sie zu diesem Zweck vorzugsweise frei von resorbierbaren, vasoaktiven Stoffen ist.

Nachfolgend wird die Erfindung anhand von Beispielen beschrieben, wobei es sich dabei um bevorzugte Ausgestaltungsformen handelt.

### Beispiel 1 (Zusammensetzung):

| | |
|---|---|
| Wasser | 87 % |
| Propylenglykol | 10 % |
| Emulgator | 2,5 % |
| Hopfenextrakt | 0,3 % |
| Olivenextrakt | 0,2 % |

### Beispiel 2 (Zusammensetzung):

| | |
|---|---|
| Wasser | 86,5 % |
| Propylenglykol | 10 % |
| Emulgator | 3 % |
| Hopfenextrakt | 0,2 % |
| Olivenextrakt | 0,3 % |

### Beispiel 3 (Zusammensetzung):

| | |
|---|---|
| Wasser | 94 % |
| Dexpanthenol | 4.5 % |
| Natürliches Konservierungsmittel | 0.5 % |
| Olivenextrakt | 0.5 % |
| Hopfenextrakt | 0,5 % |

### Beispiel 4 (Zusammensetzung):

| | |
|---|---|
| Wasser | 86.5 % |
| Propylenglykol | 10 % |
| Emulgator | 2,5 % |
| Hopfenextrakt | 0.2 % |
| Olivenextrakt | 0,3 % |
| Tomatenextrakt | 0,5 % |

### Beispiel 5 (Zusammensetzung):

| | |
|---|---|
| Wasser | 86 % |
| Propylenglykol | 10 % |
| Emulgator | 3 % |
| Hopfenextrakt | 0,3 % |
| Olivenextrakt | 0,3 % |
| Tomatenextrakt | 0,4 % |

Bei den Beispielen 1 bis 5 werden als Emulgatoren z.B. Macrogolglycerol Hydroxystearat, ethoxylierter Fettalkohol, Fettalkoholalkoxylate, Rizinusöl oder ethoxylierter Isosorbide-Dimethylether eingesetzt.

### Beispiel 6 (Formulierung für eine Nasencreme):

Wasser, Propylenglykol, Hydrogenated Arachis Hypogea, Cetyl Alkohol, Polysorbat 60, Pflanzenextrakte (z.B. 0,2 % Olivenextrakt + 0,2 % Hopfenextrakt oder 0,2 % Olivenextrakt + 0,5 % Traubenkernextrakt + 0,2 % Hopfenextrakt).

Es können auch andere Salbengrundlagen für die Herstellung einer Nasencreme mit den erwähnten Pflanzenextrakten verwendet werden.

### Beispiel 7 (Herstellung einer Nanoemulsion und einer liposomalen Formulierung):

Die Herstellung erfolgt in der Weise, dass der Emulgator und der Pflanzenextrakt gemischt werden, um eine homogene, flüssige Phase zu bilden. Dies kann sowohl bei Raumtemperatur als auch durch Erwärmen geschehen. In dieser Phase wird das Phospholipid gelöst, eventuell mit Hilfe eines Lösungsvermittlers wie z.B. Ethanol. Es entsteht eine homogene Lösung, welche mit Wasser so verdünnt wird, dass die gewünschte Konzentration des Pflanzenextraktes in Lösung erreicht wird.

## Patentansprüche

1. Pharmazeutische Zusammensetzung
- beinhaltend als Wirkstoffe Hydroxytyrosol und Xanthohumol,
- in der Form einer Formulierung für die intranasale Applikation.

2. Zusammensetzung nach Anspruch 1 zur prophylaktischen und/ oder kurativen Behandlung der Symptome von Schnupfen, insbesondere von viralem, allergischem und/ oder vasomotorischem Schnupfen.

3. Zusammensetzung nach einem der Ansprüche 1 bis 2, in der Form eines Sprays.

4. Zusammensetzung nach einem der Ansprüche 1 bis 3, **dadurch gekennzeichnet, dass** sie zusätzlich als Wirkstoffe Resveratrol und/oder Lycopin beinhaltet.

5. Zusammensetzung nach einem der Ansprüche 1 bis 4, **dadurch gekennzeichnet, dass** einer oder mehrere der Wirkstoffe biogen sind.

6. Zusammensetzung nach einem der Ansprüche 1 bis 5, **dadurch gekennzeichnet, dass** einer oder mehrere der Wirkstoffe in der Zusammensetzung als Teil von Pflanzenextrakten vorliegen,
- wobei die Konzentration des jeweiligen Wirkstoffs im jeweiligen Pflanzenextrakt vorzugsweise mindestens 5%, besonders bevorzugt mindestens 10% und insbesondere mindestens 20% bezogen auf das Gewicht des jeweiligen Pflanzenextrakts beträgt und
- wobei die Konzentration des jeweiligen Pflanzenextrakts in der Zusammensetzung jeweils vorzugsweise 0,05 bis 5%, besonders bevorzugt 0,08 bis 2% und insbesondere 0,1 bis 0,5% bezogen auf das Gewicht der Zusammensetzung beträgt.

7. Zusammensetzung nach Anspruch 6, **dadurch gekennzeichnet, dass** es sich bei den Pflanzenextrakten um einen oder mehrere der folgenden handelt: Hopfenextrakt, Olivenextrakt, Traubenextrakt, Tomatenextrakt.

8. Zusammensetzung nach einem der vorangehenden Ansprüche, **dadurch gekennzeichnet, dass** sie ein membranbildendes Molekül und einen Co-Emulgator beinhaltet.

9. Zusammensetzung nach einem der Ansprüche 1 bis 4, **dadurch gekennzeichnet, dass** sie den Wirkstoff Hydroxytyrosol als Teil von Olivenextrakt und den Wirkstoff Xanthohumol als Teil von Hopfenextrakt enthält.

10. Zusammensetzung nach Anspruch 9, **dadurch gekennzeichnet, dass** der Olivenextrakt in flüssiger Form verwendet wird.

11. Zusammensetzung nach einem der vorangehenden Ansprüche, **dadurch gekennzeichnet, dass** sie für die Anwendung bei Kindern und/oder Kleinkindern geeignet ist, wobei sie zu diesem Zweck vorzugsweise frei von resorbierbaren, vasoaktiven Stoffen ist.

## Claims

1. Pharmaceutical composition
containing hydroxytyrosol and xanthohumol as active substances,
in the form of a formulation for the intranasal application.

2. Composition according to claim 1 for the prophylactic and/or curative treatment of the symptoms of cold, in particular of viral, allergic and/or vasomotor cold.

3. Composition according to one of the claims 1 to 2 in the form of a spray.

4. Composition according to one of the claims 1 to 3, **characterized in that** it additionally contains resveratrol and/or lycopene as active substances.

5. Composition according to one of the claims 1 to 4, **characterized in that** one or several of the active substances are biogenic.

6. Composition according to one of the claims 1 to 5, **characterized in that** one or several of the active substances in the composition exist as part of plant extracts, whereby the concentration of the respective active substance in the respective plant extract is preferably at least 5%, particularly preferably at least 10% and in particular at least 20% with respect to the weight of the respective plant extract and whereby the concentration of the respective plant extract in the composition is preferably 0,05 to 5%, particularly preferably 0,08 to 2% and in particular 0,1 to 0,5% with respect to the weight of the composition.

7. Composition according to claim 6, **characterized in that** the matter is, for the plant extracts, of one or several of the following extracts: hop extract, olive extract, grape extract, tomato extract.

8. Composition according to one of the preceding claims, **characterized in that** it contains a membrane-forming molecule and a co-emulsifier.

9. Composition according to one of the claims 1 to 4, **characterized in that** it contains the active substance hydroxytyrosol as part of olive extract and the active substance xanthohumol as part of hop extract.

10. Composition according to claim 9, **characterized in that** the olive extract is used in liquid form.

11. Composition according to one of the preceding claims, **characterized in that** it is appropriate for the application for children and/or young children, whereby for this purpose it is preferably free of resorbable vasoactive substances.

## Revendications

1. Composition pharmaceutique
contenant comme substances actives de l'hydroxytyrosol et du xanthohumol, sous forme d'une formulation pour l'application intranasale.

2. Composition selon la revendication 1 pour le traitement prophylactique et/ou curatif des symptômes du rhume, en particulier du rhume viral, allergique et/ou vasomoteur.

3. Composition selon l'une des revendications 1 à 2 sous forme de spray.

4. Composition selon l'une des revendications 1 à 3, **caractérisée en ce qu'**elle contient en plus comme du resvératrol et/ou du lycopène.

5. Composition selon l'une des revendications 1 à 4, **caractérisée en ce que** l'une ou plusieurs des substances actives sont biogènes.

6. Composition selon l'une des revendications 1 à 5, **caractérisée en ce que** l'une ou plusieurs des substances actives dans la composition existent comme partie d'extraits de plantes, cependant que la concentration de la substance active respective dans l'extrait de plante respectif est de préférence au moins 5%, de manière particulièrement préférée au moins 10% et en particulier au moins 20% par rapport au poids de l'extrait de plante respectif et cependant que la concentration de l'extrait de plante respectif dans la composition est de préférence au moins 0,05 à 5%, de manière particulièrement préférée 0,08 à 2% et en particulier de 0,1 à 0,5% par rapport au poids de la composition.

7. Composition selon la revendication 6, **caractérisée en ce que** pour les extraits de plantes il s'agit d'un ou de plusieurs des extraits suivants : extrait de houblon, extrait d'olive, extrait de raisin, extrait de tomate.

8. Composition selon l'une des revendications précédentes, **caractérisée en ce qu'**elle contient une molécule qui forme une membrane et un co-émulsifiant.

9. Composition selon l'une des revendications 1 à 4, **caractérisée en ce qu'**elle contient la substance active hydroxytyrosol comme partie de l'extrait d'olive et la substance active xanthohumol comme partie de l'extrait de houblon.

10. Composition selon la revendication 9, **caractérisée en ce que** l'extrait d'olive est utilisé sous forme liquide.

11. Composition selon l'une des revendications précédentes, **caractérisée en ce qu'**elle est appropriée pour l'application chez les enfants et/ou les petits enfants, cependant que dans ce but elle est de préférence exempte de substances résorbables vasoactives.
